(19) Europäisches Patentamt
European Patent Office
Office européen des brevets

(11) EP 2 945 606 B1

(12) # EUROPEAN PATENT SPECIFICATION

(45) Date of publication and mention of the grant of the patent:
**28.11.2018 Bulletin 2018/48**

(21) Application number: **14701030.0**

(22) Date of filing: **20.01.2014**

(51) Int Cl.:
***A61K 8/41*** *(2006.01)* ***A61Q 17/04*** *(2006.01)*
***A61K 8/89*** *(2006.01)* ***A61K 8/06*** *(2006.01)*
***A61Q 19/08*** *(2006.01)*

(86) International application number:
**PCT/EP2014/051016**

(87) International publication number:
**WO 2014/111568 (24.07.2014 Gazette 2014/30)**

(54) **COSMETIC OR DERMATOLOGICAL WATER-IN-OIL EMULSION COMPRISING A MEROCYANINE AND AT LEAST ONE EMULSIFYING POLYMER OF THE POLYOXYALKYLENATED GLYCOL FATTY ACID ESTER TYPE**

KOSMETISCHE ODER DERMATOLOGISCHE WASSER-IN-ÖL-EMULSION MIT EINEM MEROCYANIN UND MINDESTENS EINEM EMULGIERENDEN POLYMER IN FORM EINES POLYOXYALKYLENIERTEN GLYCOLFETTSÄUREESTERS

ÉMULSION D'EAU DANS L'HUILE COSMÉTIQUE OU DERMATOLOGIQUE COMPRENANT UNE MÉROCYANINE ET AU MOINS UN POLYMÈRE ÉMULSIFIANT DU TYPE ESTER D'ACIDE GRAS POLYOXYALKYLÉNIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **21.01.2013 FR 1350490**

(43) Date of publication of application:
**25.11.2015 Bulletin 2015/48**

(73) Proprietor: **L'Oréal**
**75008 Paris (FR)**

(72) Inventors:
- **ROUDOT, Angelina**
  **F-94270 Le Kremlin Bicêtre (FR)**
- **CANDAU, Didier**
  **F-91570 Bievres (FR)**

(74) Representative: **Le Blainvaux Bellegarde, Françoise**
**L'Oréal**
**Service DIPI**
**9 Rue Pierre Dreyfus**
**92110 Clichy (FR)**

(56) References cited:
**WO-A1-2013/010590 WO-A2-2008/090066**
**WO-A2-2013/011094**

- **"Use of merocyanine derivatives as UV absorbers in cosmetic formulations (I)", IP.COM JOURNAL, IP.COM INC., WEST HENRIETTA, NY, US, 23 February 2009 (2009-02-23), XP013129682, ISSN: 1533-0001 cited in the application**
- **"Process for producing 3-amino-2-cyclohexan-1-ylidene compounds", IP.COM JOURNAL, IP.COM INC., WEST HENRIETTA, NY, US, 29 April 2009 (2009-04-29), XP013131313, ISSN: 1533-0001 cited in the application**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 945 606 B1

## Description

The present invention relates to a cosmetic or dermatological composition in the form of a water-in-oil emulsion comprising, in a physiologically acceptable support:

a) at least one aqueous phase, and
b) at least one oily phase, and
c) at least one merocyanine compound of formula (1) which will be defined in detail herein below and
d) at least one emulsifying system comprising at least one emulsifying polymer of the polyoxyalkylenated glycol fatty acid ester type, the said polymer is a block polymer comprising poly(hydroxylated ester) blocks and polyethylene glycol blocks, said fatty acid ester of the said emulsifying polymer as defined above bears a chain comprising from 12 to 20 carbon atoms

Another subject of the present invention consists of a composition as defined above for caring for and/or making up a keratin material, comprising the application, to the surface of the said keratin material, of said composition.

The invention also relates to a composition as defined above for limiting the darkening of the skin and/or improving the colour and/or uniformity of the complexion, comprising the application, to the surface of the keratin material, of said composition.

The invention also relates to a composition as defined above for preventing and/or treating the signs of ageing of a keratin material, comprising the application, to the surface of the keratin material, of said composition.

It is known that radiation with wavelengths of between 280 nm and 400 nm permits tanning of the human epidermis and that radiation with wavelengths of between 280 and 320 nm, known as UV-B rays, harms the development of a natural tan. Exposure is also liable to bring about a detrimental change in the biomechanical properties of the epidermis, which is reflected by the appearance of wrinkles, leading to premature ageing of the skin.

It is also known that UV-A rays with wavelengths of between 320 and 400 nm penetrate more deeply into the skin than UV-B rays. UV-A rays cause immediate and persistent browning of the skin. Daily exposure to UVA rays, even of short duration, under normal conditions can result in damage to the collagen fibres and the elastin, which is reflected by a modification in the microrelief of the skin, the appearance of wrinkles and uneven pigmentation (liver spots, lack of uniformity of the complexion).

Protection against UVA and UVB rays is thus necessary. An efficient photoprotective product should protect against both UVA and UVB radiation.

Many photoprotective compositions have been proposed to date to overcome the effects induced by UVA and/or UVB radiation. They generally contain organic or mineral UV-screening agents, which function according to their own chemical nature and according to their own properties by absorption, reflection or scattering of the UV radiation. They generally comprise mixtures of liposoluble organic screening agents and/or water-soluble UV screening agents in combination with metal oxide pigments, such as titanium dioxide or zinc oxide.

Many cosmetic compositions for limiting the darkening of the skin and improving the colour and uniformity of the complexion have been proposed to date. It is well known in the field of antisun products that such compositions may be obtained by using UV-screening agents, and in particular UVB-screening agents. Certain compositions may also contain UVA-screening agents. This screening system should cover UVB protection for the purpose of limiting and controlling the neosynthesis of melanin, which promotes the overall pigmentation, but should also cover UVA protection so as to limit and control the oxidation of the already-existing melanin leading to darkening of the skin colour.

However, not one composition contains a particular combination of UV-screening agents that would be especially suited to photoprotecting the skin and particularly to improving the quality of the skin as regards both the colour and its mechanical elasticity properties.

Advantageously, this improvement is particularly apparent on already-pigmented skin so as not to increase the melanin pigmentary load or the structure of the melanin already present in the skin.

In point of fact, the majority of the organic UV-screening agents consist of aromatic compounds which absorb in the wavelength range between 280 and 370 nm. In addition to their power for screening out sunlight, the desired photoprotective compounds should also have good cosmetic properties, good solubility in the usual solvents and in particular in fatty substances such as oils, and also good chemical stability and good photostability alone or in combination with other UV-screening agents. They should also be colourless or at least have a colour that is cosmetically acceptable to the consumer.

One of the main drawbacks known to date of these compositions is that these screening systems insufficiently protect against UV rays and in particular against long UVA rays with wavelengths beyond 370 nm, for the purpose of controlling photo-induced pigmentation and its evolution by means of a system for screening out UV over the entire UV spectrum.

Among all the compounds that have been recommended for this purpose an advantageous family of UV-screening agents has been proposed, which consists of carbon-bearing merocyanine derivatives, which is described in patent US 4 195 999, patent application WO 2004/006 878, patent applications WO2008/090066, WO2011 /113718, WO2009/027258, WO2013/010590, WO2013/011094, WO20130/11480 and the documents IP COM JOURNAL N°000179675D published on February 23, 2009, IP COM JOURNAL N°000182396D published on April 29, IP COM JOURNAL N° 000189542D published on November 12, 2009, IP COM Journal N°IPCOM000011179D published on 03/04/2004. Some of these compounds may show the following drawbacks :

- relatively unsatisfactory solubility in the usual solvents and in particular in fatty substances such as oils which may require a laborious formulation process and/or may result in cosmetic drawbacks such as a greasy effect on application ;
- an unsatisfactory chemical stability and/or unsatisfactory photostability
- produce a color liable to discourage the consumer from using a cosmetic or dermatological composition containing them.

Antisun compositions are quite often in the form of an emulsion of oil-in-water type (i.e. a cosmetically acceptable support consisting of a continuous aqueous dispersing phase and of a discontinuous oily dispersed phase) or of the water-in-oil type (i.e. a cosmetically acceptable support consisting of a continuous oily dispersing phase and of a discontinuous aqueous dispersed phase) which contains, in varying concentrations, one or more conventional lipophilic and/or hydrophilic organic screening agents which are capable of selectively absorbing harmful UV rays, these screening agents (and the amounts thereof) being selected as a function of the desired sun protection factor.

The Applicant has found in the course of its research that some of the merocyanines could lose their efficacy in the presence of certain particular emulsifiers in antisun compositions. The Applicant has especially observed that some of these merocyanines degrade chemically in direct emulsions in the presence of certain emulsifying systems as those constituted of at least one alkali metal salt of a phosphoric acid ester of a fatty alcohol.

The need thus remains to find novel emulsions that ensure a good chemical stability of merocyanines without the drawbacks as previously defined.

The Applicant has discovered, surprisingly, that this objective can be achieved by using a specific merocyanine of formula (1) which will be defined herein below in a water-in-oil emulsion support containing at least one polymer of the polyoxyalkylenated glycol fatty acid ester type, without affecting the properties of screening out the long UVA radiation of these merocyanines.

Furthermore, the merocyanine compounds of formula (1) herein below, present surprinsingly the advantage to be significantly less colored than the merocyanine compounds as disclosed in the application WO2008/090066 as the compound MC11 also called MC03 in the application WO2009/027258.

Those discoveries form the basis of the present invention.

Thus, in accordance with one of the objects of the present invention, a cosmetic or dermatological composition in the form of a water-in-oil emulsion is now proposed, comprising, in a physiologically acceptable support:

a) at least one aqueous phase, and
b) at least one oily phase, and
c) at least one merocyanine compound of formula (1) defined hereinbelow in detail and
d) at least one emulsifying system comprising at least one emulsifying polymer of the polyoxyalkylenated glycol fatty acid ester type, said polymer is a block polymer comprising poly(hydroxylated ester) blocks and polyethylene glycol blocks, said fatty acid ester of the said emulsifying polymer as defined above bears a chain comprising from 12 to 20 carbon atoms

Another subject of the present invention consists of a composition as defined above for caring for and/or making up a keratin material, comprising the application, to the surface of the said keratin material, of the said composition.

The invention also relates to a composition as defined above for limiting the darkening of the skin and/or improving the colour and/or uniformity of the complexion, comprising the application, to the surface of the keratin material, of the said composition.

The invention also relates to a composition as defined above for preventing and/or treating the signs of ageing of a keratin material, comprising the application, to the surface of the keratin material, of the said composition.

Other characteristics, aspects and advantages of the invention will emerge on reading the detailed description that follows.

The expression "human keratin materials" means the skin (body, face, area around the eyes), hair, eyelashes, eyebrows, body hair, nails, lips or mucous membranes.

The term "physiologically acceptable" means compatible with the skin and/or its integuments, having a pleasant colour, odour and feel and not causing any unacceptable discomfort (stinging, tautness or redness) liable to discourage

the consumer from using this composition.

The term "between X and Y" means the range of values also including the limits X and Y.

According to the invention, the term "preventing" or "prevention" means reducing the risk of occurrence or slowing down the occurrence of a given phenomenon, namely, according to the present invention, the signs of ageing of a keratin material.

The term "water-in-oil emulsion" means any macroscopically homogeneous, kinetically stable composition comprising at least two mutually immiscible phases; one being the dispersing continuous oily phase and the other being the aqueous phase dispersed in the said continuous oily phase in the form of droplets. The two phases are kinetically stabilized by at least one emulsifying system generally comprising at least one emulsifying surfactant.

Emulsions are distinguished as being of the oil-in-water type, known as "direct" emulsions, consisting of an aqueous dispersing continuous phase and of an oily dispersed discontinuous phase, and emulsions of the water-in-oil type, known as inverse emulsions, consisting of an oily dispersing continuous phase and of an aqueous dispersed discontinuous phase. There are also multiple emulsions, for instance water-in-oil-in-water or oil-in-water-in-oil emulsions.

The term "emulsifying system" refers to any compound or mixture of compounds that is capable of increasing the kinetic stability of an emulsion. These compounds are generally amphiphilic and are surfactants characterized by their more or less hydrophilic or more or less lipophilic nature which will determine their ability to stabilize direct emulsions or inverse emulsions. They are especially classified by their HLB according to the calculation method of W.C. Griffin in the document "Classification of Surface Active Agents by HLB, Journal of the Society of Cosmetic Chemists 1 (1949) 311" and in the document "Calculation of HLB of Non Ionic Surfactants, Journal of the Society of Cosmetic Chemists 5 (1954) 249". The calculation of the HLB according to this calculation method is performed according to the equation:

$$HLB = 20 \text{ X } Mh/M$$

where Mh is the molar mass of the hydrophilic part of the surfactant and M is the total molecular mass of the molecule.

The term "emulsifying polymer" means any polymer with emulsifying properties thus making it possible to increase the kinetic stability of an emulsion. This polymer is generally amphiphilic and is a surfactant characterized by its more or less hydrophilic or more or less lipophilic nature which will determine its ability to stabilize direct emulsions or inverse emulsions. It is classified especially by its HLB value according to the method indicated above.

For the purposes of the invention, the term "polymer" means a compound containing at least two repeating units, preferably at least three repeating units and better still ten repeating units.

**MEROCYANINES**

According to the present invention, the merocyanine compounds in accordance with the invention correspond to formula (1) below, and also the E/E- or E/Z-geometrical isomer forms thereof:

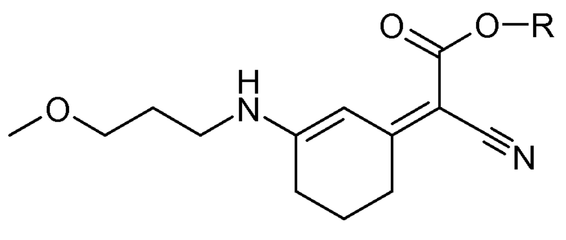

(1)

in which:

R is a $C_1$-$C_{22}$ alkyl group, a $C_2$-$C_{22}$ alkenyl group, a $C_2$-$C_{22}$ alkynyl group, a $C_3$-$C_{22}$ cycloalkyl group or a $C_3$-$C_{22}$ cycloalkenyl group, the said groups possibly being interrupted with one or more O.

The merocyanine compounds of the invention may be in their E/E- or E/Z-geometrical isomer forms.

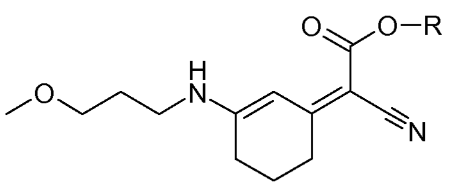

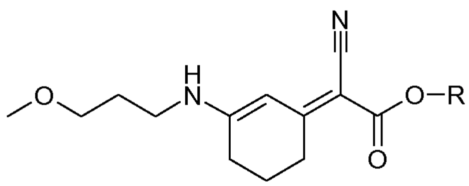

The preferential compounds of formula (1) are those in which:
R is a $C_1$-$C_{22}$ alkyl, which may be interrupted with one or more O.

Among the compounds of formula (1), use will be made more particularly of those chosen from the following compounds, and also the E/E- or E/Z- geometrical isomer forms thereof:

| | | | |
|---|---|---|---|
| 1 | 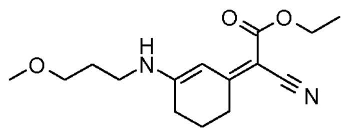 ethyl (2Z)-cyano{3-[(3-methoxypropyl)amino] cyclohex-2-en-1-ylidene}ethanoate | 4 | 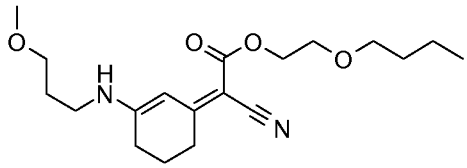 2-butoxyethyl (2Z)-cyano{3-[(3-methoxypropyl) amino]cyclohex-2-en-1-ylidene}ethanoate |
| 2 | 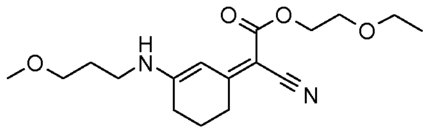 2-ethoxyethyl (2Z)-cyano{3-[(3-methoxypropyl) amino]cyclohex-2-en-1-ylidene}ethanoate | 5 | 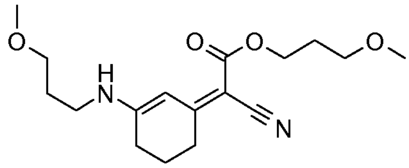 3-methoxypropyl (2Z)-cyano{3-[(3-methoxypropyl) amino]cyclohex-2-en-1-ylidene}ethanoate |
| 3 | 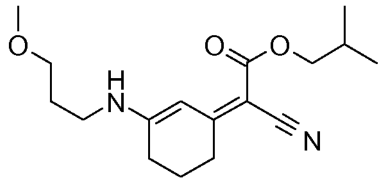 2-methylpropyl (2Z)-cyano{3-[(3-methoxypropyl) amino]cyclohex-2-en-1-ylidene}ethanoate | 6 | 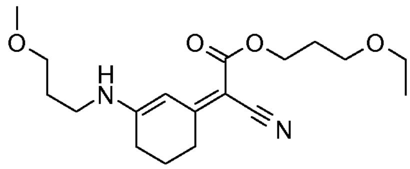 3-ethoxypropyl (2Z)-cyano{3-[(3-methoxypropyl) amino]cyclohex-2-en-1-ylidene}ethanoate |

According to a more particularly preferred mode of the invention, use will be made of the compound 2-ethoxyethyl (2Z)-cyano{3-[(3-methoxypropyl)amino]cyclohex-2-en-1-ylidene}ethanoate (2) in its E/Z geometrical configuration having the following structure:

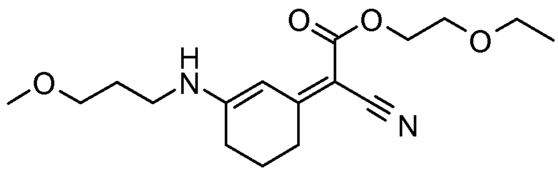

and/or in its E/E geometrical configuration having the following structure:

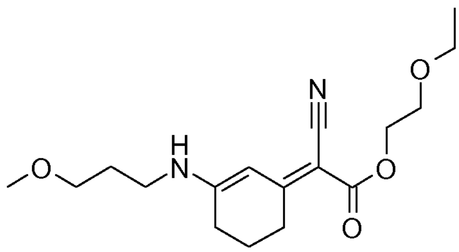

The merocyanines of formula (1) according to the invention are preferably present in the compositions according to the invention in a concentration ranging from 0.1% to 10% by weight and preferentially from 0.2% to 5% by weight relative to the total weight of the composition.

The compounds of formula (1) may be prepared according to the protocols described in Pat. Appl. WO 2007/071

582, in IP.com Journal (2009), 9(5A), 29-30 IPCOM000182396D under the title "Process for producing 3-amino-2-cyclohexan-1-ylidehe compounds" and in US-A-4 749 643 on column 13, line 66 - column 14, line 57 and the references cited in this regard.

## EMULSIFYING SYSTEM

### Emulsifying polymer of the palyoxyalkylenated glycol fatty acid ester type

A second essential compound of the compositions according to the invention is an emulsifying polymer of the polyoxyalkylenated glycol fatty acid ester types, aid polymer is a block polymer comprising poly(hydroxylated ester) blocks and polyethylene glycol blocks, said fatty acid ester of the said emulsifying polymer as defined above bears a chain comprising from 12 to 20 carbon atoms-

The fatty acid ester of the said polymer is preferably polyhydroxylated. In particular, this polymer is a block polymer, preferably of structure ABA, comprising poly(hydroxylated ester) blocks and polyethylene glycol blocks.

The fatty acid ester of the said emulsifying polymer as defined above generally bears a chain comprising from 12 to 20 carbon atoms and preferably from 14 to 18 carbon atoms. The esters may be chosen especially from the oleates, palmitates and stearates.

The polyethylene glycol blocks of the said emulsifying polymer as defined above preferably comprise from 4 to 50 mol of ethylene oxide and more preferably from 20 to 40 mol of ethylene oxide.

A compound that is particularly suitable for producing the compositions of the invention is the 30 OE polyethylene glycol di-polyhydroxystearate sold under the trade name Arlacel P 135 by the company ICI.

The emulsifying polymer of the polyoxyalkylenated glycol fatty acid ester type is preferably present in the compositions according to the invention in a content ranging from 0.2% to 10% by weight relative to the total weight of the composition and more preferentially from 0.25% to 5% by weight relative to the total weight of the composition.

### Additional emulsifying agents

As other emulsifying surfactants that may be used for the preparation of the water-in-oil emulsions of the invention, examples that may be mentioned include sorbitan alkyl esters or ethers, glycerol alkyl esters or ethers other than the polyoxyalkylenated glycol fatty acid ester polymers of the invention, or sugar alkyl esters or ethers; silicone surfactants, for instance dimethicone copolyols, such as the mixture of cyclomethicone and of dimethicone copolyol, sold under the name DC 5225 C by the company Dow Corning, and alkyldimethicone copolyols such as laurylmethicone copolyol sold under the name Dow Corning 5200 Formulation Aid by the company Dow Corning; cetyldimethicone copolyol, such as the product sold under the name Abil EM 90R by the company Goldschmidt, and the mixture of cetyldimethicone copolyol, of polyglyceryl isostearate (4 mol) and of hexyl laurate, sold under the name Abil WE 09 by the company Goldschmidt. One or more coemulsifiers may also be added thereto, which may be chosen advantageously from the group comprising polyol alkyl esters.

Examples of additional glycerol and/or sorbitan esters that may be mentioned include polyglyceryl isostearate, such as the product sold under the name Isolan Gl 34 by the company Goldschmidt; sorbitan isostearate, such as the product sold under the name Arlacel 987 by the company ICI; sorbitan glyceryl isostearate, and such as the product sold under the name Arlacel 986 by the company ICI, and mixtures thereof.

## OILY PHASE

The compositions in accordance with the invention comprise at least one oily phase.

For the purposes of the invention, the term "oily phase" means a phase comprising at least one oil and all of the liposoluble and lipophilic ingredients and the fatty substances used for the formulation of the compositions of the invention.

The term "oil" means any fatty substance which is in liquid form at room temperature (20 - 25°C) and at atmospheric pressure (760 mmHg).

An oil that is suitable for use in the invention may be volatile or non-volatile.

An oil that is suitable for use in the invention may be chosen from hydrocarbon-based oils, silicone oils and fluoro oils, and mixtures thereof.

A hydrocarbon-based oil that is suitable for use in the invention may be an animal hydrocarbon-based oil, a plant hydrocarbon-based oil, a mineral hydrocarbon-based oil or a synthetic hydrocarbon-based oil.

An oil that is suitable for use in the invention may be advantageously chosen from mineral hydrocarbon-based oils, plant hydrocarbon-based oils, synthetic hydrocarbon-based oils and silicone oils, and mixtures thereof.

For the purposes of the present invention, the term "silicone oil" means an oil comprising at least one silicon

atom, and especially at least one Si-O group.

The term "hydrocarbon-based oil" means an oil comprising mainly hydrogen and carbon atoms.

The term "fluoro oil" means an oil comprising at least one fluorine atom.

A hydrocarbon-based oil that is suitable for use in the invention may also optionally comprise oxygen, nitrogen, sulfur and/or phosphorus atoms, for example in the form of hydroxyl, amine, amide, ester, ether or acid groups, and in particular in the form of hydroxyl, ester, ether or acid groups.

The oily phase generally comprises, in addition to the lipophilic UV-screening agent(s), at least one volatile or non-volatile hydrocarbon-based oil and/or one volatile and/or non-volatile silicone oil.

For the purposes of the invention, the term "volatile oil" means an oil that is capable of evaporating on contact with the skin or the keratin fibre in less than one hour, at room temperature and atmospheric pressure. The volatile oil(s) of the invention are volatile cosmetic oils which are liquid at room temperature and which have a non-zero vapour pressure, at room temperature and atmospheric pressure, ranging in particular from 0.13 Pa to 40 000 Pa ($10^{-3}$ to 300 mmHg), in particular ranging from 1.3 Pa to 13 000 Pa (0.01 to 100 mmHg) and more particularly ranging from 1.3 Pa to 1300 Pa (0.01 to 10 mmHg).

The term "non-volatile oil" means an oil which remains on the skin or the keratin fibre, at room temperature and atmospheric pressure, for at least several hours and which in particular has a vapour pressure of less than $10^{-3}$ mmHg (0.13 Pa).

Hydrocarbon-based oils

As non-volatile hydrocarbon-based oils that may be used according to the invention, mention may be made especially of:

(i) hydrocarbon-based oils of plant origin, such as glyceride triesters, which are generally triesters of fatty acids and of glycerol, the fatty acids of which can have varied chain lengths from $C_4$ to $C_{24}$, it being possible for these chains to be saturated or unsaturated and linear or branched; these oils are in particular wheat germ oil, sunflower oil, grape seed oil, sesame oil, corn oil, apricot oil, castor oil, shea oil, avocado oil, olive oil, soybean oil, sweet almond oil, palm oil, rapeseed oil, cottonseed oil, hazelnut oil, macadamia oil, jojoba oil, alfalfa oil, poppy oil, pumpkin seed oil, marrow oil, blackcurrant oil, evening primrose oil, millet oil, barley oil, quinoa oil, rye oil, safflower oil, candlenut oil, passionflower oil and musk rose oil; or also caprylic/capric acid triglycerides, such as those sold by Stéarineries Dubois or those sold under the names Miglyol 810®, 812® and 818® by Dynamit Nobel;

(ii) synthetic ethers containing from 10 to 40 carbon atoms;

(iii) linear or branched hydrocarbons of mineral or synthetic origin, such as petroleum jelly, polydecenes, hydrogenated polyisobutene such as Parleam, and squalane, and mixtures thereof;

(iv) synthetic esters, for instance oils of formula RCOOR' in which R represents a linear or branched fatty acid residue containing from 1 to 40 carbon atoms and R' represents a hydrocarbon-based chain that is especially branched, containing from 1 to 40 carbon atoms on condition that
R + R' is $\geq$10, for instance Purcellin oil (cetearyl octanoate), isopropyl myristate, isopropyl palmitate, $C_{12}$-$C_{15}$ alkyl benzoate, such as the product sold under the trade name Finsolv TN® or Witconol TN® by Witco or Tegosoft TN® by Evonik Goldschmidt, 2-ethylphenyl benzoate, such as the commercial product sold under the name X-Tend 226® by ISP, isopropyl lanolate, hexyl laurate, diisopropyl adipate, isononyl isononanoate, oleyl erucate, 2-ethylhexyl palmitate, isostearyl isostearate, diisopropyl sebacate, such as the product sold under the name of "Dub Dis" by Stearinerie Dubois, octanoates, decanoates or ricinoleates of alcohols or polyalcohols, such as propylene glycol dioctanoate; hydroxylated esters, such as isostearyl lactate or diisostearyl malate; and pentaerythritol esters; citrates or tartrates, such as di(linear $C_{12}$-$C_{13}$ alkyl) tartrates, such as those sold under the name Cosmacol ETI® by Enichem Augusta Industriale, and also di(linear $C_{14}$-$C_{15}$ alkyl) tartrates, such as those sold under the name Cosmacol ETL® by the same company; or acetates;

(v) fatty alcohols that are liquid at room temperature, containing a branched and/or unsaturated carbon-based chain containing from 12 to 26 carbon atoms, for instance octyldodecanol, isostearyl alcohol, oleyl alcohol, 2-hexyldecanol, 2-butyloctanol or 2-undecylpentadecanol;

(vi) higher fatty acids, such as oleic acid, linoleic acid or linolenic acid;

(vii) carbonates, such as dicaprylyl carbonate, such as the product sold under the name Cetiol CC® by Cognis;

(viii) fatty amides, such as isopropyl N-lauroyl sarcosinate, such as the product sold under the trade name Eldew SL 205® from Ajinomoto;

and mixtures thereof.

Preference will more particularly be given, among the non-volatile hydrocarbon-based oils that may be used according to the invention, to glyceride triesters and in particular to caprylic/capric acid triglycerides, synthetic esters and in particular isononyl isononanoate, oleyl erucate, $C_{12}$-$C_{15}$ alkyl benzoate, 2-ethylphenyl benzoate and fatty alcohols, in particular octyldodecanol.

Mention may in particular be made, as volatile hydrocarbon-based oils that may be used according to the invention, of hydrocarbon-based oils containing from 8 to 16 carbon atoms and in particular of branched $C_8$-$C_{16}$ alkanes, such as $C_8$-$C_{16}$ isoalkanes of petroleum origin (also known as isoparaffins), such as isododecane (also known as 2,2,4,4,6-pentamethylheptane), isodecane or isohexadecane, the oils sold under the Isopar or Permethyl trade names, branched $C_8$-$C_{16}$ esters, isohexyl neopentanoate, and mixtures thereof.

Mention may also be made of the alkanes described in Cognis patent applications WO 2007/068 371 or WO 2008/155 059 (mixtures of distinct alkanes differing by at least one carbon). These alkanes are obtained from fatty alcohols, which are themselves obtained from coconut or palm oil. Mention may be made of the mixtures of n-undecane ($C_{11}$) and n-tridecane ($C_{13}$) obtained in Examples 1 and 2 of patent application WO 2008/155 059 from the company Cognis. Mention may also be made of n-dodecane ($C_{12}$) and n-tetradecane ($C_{14}$) sold by Sasol under the respective references Parafol 12-97 and Parafol 14-97®, and also mixtures thereof.

Use may also be made of other volatile hydrocarbon-based oils, such as petroleum distillates, in particular those sold under the name Shell Solt® by Shell. According to one embodiment, the volatile solvent is chosen from volatile hydrocarbon-based oils containing from 8 to 16 carbon atoms, and mixtures thereof.

<u>b) Silicone oils</u>

The non-volatile silicone oils may be chosen in particular from non-volatile polydimethylsiloxanes (PDMSs), polydimethylsiloxanes comprising alkyl or alkoxy groups which are pendent and/or at the end of the silicone chain, which groups each contain from 2 to 24 carbon atoms, or phenyl silicones, such as phenyl trimethicones, phenyl dimethicones, phenyl(trimethylsiloxy)diphenylsiloxanes, diphenyl dimethicones, diphenyl(methyldiphenyl)trisiloxanes or (2-phenylethyl)trimethylsiloxysilicates.

Examples of volatile silicone oils that may be mentioned include volatile linear or cyclic silicone oils, especially those with a

viscosity $\leq$ 8 centistokes ($8 \times 10^{-6}$ $m^2/s$) and especially containing from 2 to 7 silicon atoms, these silicones optionally comprising alkyl or alkoxy groups containing from 1 to 10 carbon atoms. As volatile silicone oils that may be used in the invention, mention may be made especially of octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane and dodecamethylpentasiloxane, and mixtures thereof.

Mention may also be made of the volatile linear alkyltrisiloxane oils of general formula (I):

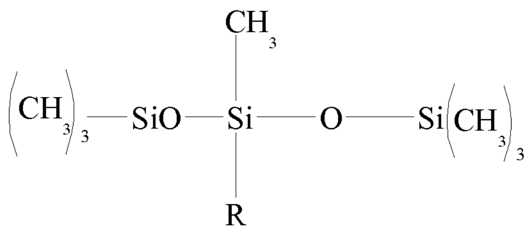

where R represents an alkyl group comprising from 2 to 4 carbon atoms, one or more hydrogen atoms of which may be substituted with a fluorine or chlorine atom.

Among the oils of general formula (I), mention may be made of:

3-butyl-1,1,1,3,5,5,5-heptamethyltrisiloxane,
3-propyl-1,1,1,3,5,5,5-heptamethyltrisiloxane, and
3-ethyl-1,1,1,3,5,5,5-heptamethyltrisiloxane,

corresponding to the oils of formula (I) for which R is, respectively, a butyl group, a propyl group or an ethyl group.

Fluoro oils

Use may also be made of volatile fluoro oils, such as nonafluoromethoxybutane, decafluoropentane, tetradecafluorohexane, dodecafluoropentane, and mixtures thereof.
An oily phase according to the invention may also comprise other fatty substances, mixed with or dissolved in the oil.
Another fatty substance that may be present in the oily phase may be, for example:

- a fatty acid chosen from fatty acids comprising from 8 to 30 carbon atoms, such as stearic acid, lauric acid, palmitic acid and oleic acid;
- a wax chosen from waxes such as lanolin, beeswax, carnauba or candelilla wax, paraffin waxes, lignite waxes, microcrystalline waxes, ceresin or ozokerite, or synthetic waxes, such as polyethylene waxes or Fischer-Tropsch waxes;
- a gum chosen from silicone gums (dimethiconol);
- a pasty compound, such as polymeric or non-polymeric silicone compounds, esters of a glycerol oligomer, arachidyl propionate, fatty acid triglycerides and derivatives thereof;
- and mixtures thereof.

According to a particular form of the invention, the overall oily phase, including all the lipophilic substances of the composition that are capable of being dissolved in this same phase, represents from 5% to 95% by weight and preferably from 10% to 80% by weight, relative to the total weight of the composition.

**AQUEOUS PHASE**

The compositions according to the invention comprise at least one aqueous phase.
The aqueous phase contains water, and optionally other water-soluble or water-miscible organic solvents.
An aqueous phase that is suitable for use in the invention may comprise, for example, a water chosen from a water from a natural source, such as water from La Roche-Posay, water from Vittel or water from Vichy, or a floral water.
The water-soluble or water-miscible solvents that are suitable for use in the invention comprise short-chain monoalcohols, for example $C_1$-$C_4$ monoalcohols such as ethanol and isopropanol; diols or polyols, for instance ethylene glycol, 1,2-propylene glycol, 1,3-butylene glycol, hexylene glycol, diethylene glycol, dipropylene glycol, 2-ethoxyethanol, diethylene glycol monomethyl ether, triethylene glycol monomethyl ether, glycerol and sorbitol, and mixtures thereof.
According to a preferred embodiment, use may be made more particularly of ethanol, propylene glycol or glycerol, and mixtures thereof.
Preferentially, the overall aqueous phase, including all the hydrophilic substances of the composition that are capable of being dissolved in this same phase, represents from 5% to 95% by weight and preferentially from 10% to 80% by weight relative to the total weight of the composition.

**ADDITIVES**

**Additional UV-screening agents**

The compositions according to the invention may also contain one or more additional UV-screening agents chosen from hydrophilic, lipophilic or insoluble organic UV-screening agents and/or one or more mineral pigments. Preferentially, it will consist of at least one hydrophilic, lipophilic or insoluble organic UV-screening agent.
The term "hydrophilic UV-screening agent" means any cosmetic or dermatological organic or mineral compound for screening out UV radiation which is capable of being fully dissolved in molecular form in a liquid aqueous phase or of being dissolved in colloidal form (for example in micellar form) in a liquid aqueous phase.
The term "lipophilic screening agent" means any cosmetic or dermatological organic or mineral compound for screening out UV radiation, which can be fully dissolved in molecular state in a liquid fatty phase or which can be dissolved in colloidal form (for example in micellar form) in a liquid fatty phase.
The term "insoluble UV-screening agent" means any cosmetic or dermatological organic or mineral compound for screening out UV radiation which has a water-solubility of less than 0.5% by weight and a solubility of less than 0.5% by weight in the majority of organic solvents such as liquid paraffin, fatty alkyl benzoates and fatty acid triglycerides, for example Miglyol 812® sold by the company Dynamit Nobel. This solubility, determined at 70°C, is defined as the amount of product in solution in the solvent at equilibrium with an excess of solid in suspension after returning to room temperature. It may be readily evaluated in the laboratory.
The additional organic UV-screening agents are chosen in particular from cinnamic compounds; anthranilate

compounds; salicylic compounds; dibenzoylmethane compounds; benzylidenecamphor compounds; benzophenone compounds; β,β-diphenylacrylate compounds; triazine compounds; benzotriazole compounds; benzalmalonate compounds, in particular those cited in patent US 5 624 663; benzimidazole derivatives; imidazoline compounds; bis-benzoazolyl compounds, as described in patents EP 669 323 and US 2,463,264; p-aminobenzoic acid (PABA) compounds; methylenebis(hydroxyphenylbenzotriazole) compounds, such as described in patent applications US 5 237 071, US 5 166 355, GB 2 303 549, DE 197 26 184 and EP 893 119; benzoxazole compounds, as described in patent applications EP 0 832 642, EP 1 027 883, EP 1 300 137 and DE 101 62 844; screening polymers and screening silicones, such as those described in particular in patent application WO 93/04665; α-alkylstyrene-based dimers, such as those described in patent application DE 198 55 649; 4,4-diarylbutadiene compounds, as described in patent applications EP 0 967 200, DE 197 46 654, DE 197 55 649, EP-A-1 008 586, EP 1 133 980 and EP 133 981, and mixtures thereof.

As examples of organic photoprotective agents, mention may be made of those denoted hereinbelow under their INCI name:

Cinnamic compounds:

Ethylhexyl Methoxycinnamate, sold in particular under the trade name Parsol MCX® by DSM Nutritional Products,
Isopropyl Methoxycinnamate,
Isoamyl p-methoxycinnamate sold under the trade name Neo Heliopan E 1000® by Symrise,
DEA Methoxycinnamate,
Diisopropyl Methyl Cinnamate,
Glyceryl Ethylhexanoate Dimethoxycinnamate.

Dibenzoylmethane compounds:

Butylmethoxydibenzoylmethane sold in particular under the trade name Parsol 1789® by DSM Nutritional Products,
Isopropyldibenzoylmethane.

para-Aminobenzoic compounds:

PABA,
Ethyl PABA,
Ethyl Dihydroxypropyl PABA,
Ethylhexyl Dimethyl PABA, sold in particular under the name Escalol 507® by ISP, Glyceryl PABA,
PEG-25 PABA, sold under the name Uvinul P 25® by BASF.

Salicylic compounds:

Homosalate, sold under the name Eusolex HMS® by Rona/EM Industries, Ethylhexyl Salicylate, sold under the name Neo Heliopan OS® by Symrise, Dipropylene Glycol Salicylate, sold under the name Dipsal® by Scher,
TEA Salicylate, sold under the name Neo Heliopan TS® by Symrise.

β,β-Diphenylacrylate compounds:

Octocrylene, sold in particular under the trade name Uvinul N 539® by BASF, Etocrylene, sold in particular under the trade name Uvinul N 35® by BASF.

Benzophenone compounds:

Benzophenone-1, sold under the trade name Uvinul 400® by BASF,
Benzophenone-2, sold under the trade name Uvinul D 50® by BASF,
Benzophenone-3 or Oxybenzone, sold under the trade name Uvinul M 40® by BASF,
Benzophenone-4, sold under the trade name Uvinul MS 40® by BASF, Benzophenone-5,
Benzophenone-6, sold under the trade name Helisorb 11® by Norquay,
Benzophenone-8, sold under the trade name Spectra-Sorb UV-240 by American Cyanamid,

Benzophenone-9, sold under the trade name Uvinul DS 49® by BASF, Benzophenone-12,

n-Hexyl 2-(4-diethylamino-2-hydroxybenzoyl)benzoate, sold under the trade name Uvinul A Plus® or, as a mixture with octyl methoxycinnamate, under the trade name Uvinul A Plus B® by BASF,
1,1'-(1,4-Piperazinediyl)bis[1-[2-[4-(diethylamino)-2-hydroxybenzoyl]phenyl]methanone] (CAS 919803-06-8), such as described in patent application WO 2007/071 584; this compound advantageously being used in micronized form (mean size of 0.02 to 2 μm), which may be obtained, for example, according to the micronization process described in patent applications GB-A-2 303 549 and EP-A-893 119, and in particular in the form of an aqueous dispersion.

Benzylidenecamphor compounds:

3-Benzylidene Camphor, manufactured under the name Mexoryl SD® by Chimex, 4-Methylbenzylidene Camphor, sold under the name Eusolex 6300® by Merck, Benzylidene Camphor Sulfonic Acid, manufactured under the name Mexoryl SL® by Chimex,
Camphor Benzalkonium Methosulfate, manufactured under the name Mexoryl SO® by Chimex,
Terephthalylidene Dicamphor Sulfonic Acid, manufactured under the name Mexoryl SX® by Chimex,
Polyacrylamidomethyl Benzylidene Camphor, manufactured under the name Mexoryl SW® by Chimex.

Phenylbenzimidazole compounds:

Phenylbenzimidazole Sulfonic Acid, sold in particular under the trade name Eusolex 232® by Merck.

Bis-benzazolyl compounds:

Disodium Phenyl Dibenzimidazole Tetrasulfonate, sold under the trade name Neo Heliopan AP® by Haarmann and Reimer.

Phenylbenzotriazole compounds:

Drometrizole Trisiloxane, sold under the name Silatrizole® by Rhodia Chimie.

Methylenebis(hydroxylphenylbenzotriazole) compounds:

Methylenebis(benzotriazolyl)tetramethylbutylphenol especially in solid form, for instance the product sold under the trade name Mixxim BB/100® by Fairmount Chemical or in the form of an aqueous dispersion of micronized particles with a mean particle size ranging from 0.01 to 5 μm, more preferentially from 0.01 to 2 μm and more particularly from 0.020 to 2 μm with at least one alkylpolyglycoside surfactant of structure $C_nH_{2n+1}O(C_6H_{10}O_5)_xH$, in which n is an integer from 8 to 16 and x is the mean degree of polymerization of the $(C_6H_{10}O_5)$ unit and ranges from 1.4 to 1.6, such as described in patent GB-A-2 303 549, sold in particular under the trade name Tinosorb M® by BASF, or in the form of an aqueous dispersion of micronized particles with a mean particle size ranging from 0.02 to 2 μm, more preferably from 0.01 to 1.5 μm and more particularly from 0.02 to 1 μm, in the presence of at least one polyglyceryl mono($C_8$-$C_{20}$)alkyl ester with a degree of glycerol polymerization of at least 5, such as the aqueous dispersions described in patent application WO 2009/063 392.

Triazine compounds:

- Bis-Ethylhexyloxyphenol Methoxyphenyl Triazine, sold under the trade name Tinosorb S® by BASF,
- Ethylhexyl Triazone, sold in particular under the trade name Uvinul T150® by BASF,
- Diethylhexyl Butamido Triazone, sold under the trade name Uvasorb HEB® by Sigma 3V,
- 2,4,6-tris(dineopentyl 4'-aminobenzalmalonate)-s-triazine,
- 2,4,6-tris(diisobutyl 4'-aminobenzalmalonate)-s-triazine;
- 2,4-bis(n-butyl 4'-aminobenzoate)-6-(aminopropyltrisiloxane)-s-triazine,
- 2,4-bis(dineopentyl 4'-aminobenzalmalonate)-6-(n-butyl 4'-aminobenzoate)-s-triazine,
- symmetrical triazine screening agents substituted with naphthalenyl groups or polyphenyl groups described in patent US 6 225 467, patent application WO 2004/085 412 (see compounds 6 and 9) or the document "Symmetrical Triazine Derivatives", IP.COM IPCOM000031257 Journal, INC, West Henrietta, NY, US (20 September 2004), in particular

2,4,6-tris(diphenyl)triazine and 2,4,6-tris(terphenyl)triazine, which is also mentioned in patent applications WO 06/035 000, WO 06/034 982, WO 06/034 991, WO 06/035 007, WO 2006/034 992 and WO 2006/034 985, these compounds advantageously being used in micronized form (mean particle size of 0.02 to 3 μm), which may be obtained, for example, according to the micronization process described in patent applications GB-A-2 303 549 and EP-A-893 119, and in particular in the form of an aqueous dispersion;

- silicone triazines substituted with two aminobenzoate groups, as described in patent EP 0 841 341, in particular 2,4-bis(n-butyl 4'-aminobenzalmalonate)-6-[(3-{1,3,3,3-tetramethyl-1-[(trimethylsilyl)oxy]disiloxanyl}propyl)amino]-s-triazine.

Anthranilic compounds:

Menthyl Anthranilate, sold under the trade name Neo Heliopan MA® by Symrise.

Imidazoline compounds:

Ethylhexyl dimethoxybenzylidene dioxoimidazoline propionate.

Benzalmalonate compounds:

Polyorganosiloxane comprising benzalmalonate functional groups, such as Polysilicone-15, sold under the trade name Parsol SLX® by Hoffmann-LaRoche.

4,4-Diarylbutadiene compounds:

1,1-Dicarboxy(2,2'-dimethylpropyl)-4,4-diphenylbutadiene.

Benzoxazole compounds:

2,4-Bis[4-[5-(1,1-dimethylpropyl)benzoxazol-2-yl]phenylimino]-6-[(2-ethylhexyl)imino]-1,3,5-triazine, sold under the name of Uvasorb K2A® by Sigma 3V.

The preferred organic screening agents are chosen from:

Ethylhexyl Methoxycinnamate,
Ethylhexyl salicylate,
Homosalate,
Butyl Methoxy Dibenzoylmethane,
Octocrylene,
Phenylbenzimidazole Sulfonic Acid,
Benzophenone-3,
Benzophenone-4,
Benzophenone-5,
n-Hexyl 2-(4-diethylamino-2-hydroxybenzoyl)benzoate,
4-Methylbenzylidene Camphor,
Terephthalylidene Dicamphor Sulfonic Acid,
Disodium Phenyl Dibenzimidazole Tetrasulfonate,
Methylene Bis-Benzotriazolyl Tetramethylbutylphenol,
Bis-Ethylhexyloxyphenyl Methoxyphenyl Triazine,
Ethylhexyl Triazone,
Diethylhexyl Butamido Triazone,
2,4,6-Tris(dineopentyl 4'-aminobenzalmalonate)-s-triazine,
2,4,6-Tris(diisobutyl 4'-aminobenzalmalonate)-s-triazine,
2,4-bis(n-butyl 4'-aminobenzoate)-6-(aminopropyltrisiloxane)-s-triazine,
2,4-Bis(dineopentyl 4'-aminobenzalmalonate)-6-(n-butyl 4'-aminobenzoate)-s-triazine,
2,4-Bis(n-butyl 4'-aminobenzalmalonate)-6-[(3-{1,3,3,3-tetramethyl-1-[(trimethylsilyl)oxy]disiloxanyl}propyl)amino]-s-triazine,
2,4,6-Tris(diphenyl)triazine,
2,4,6-Tris(terphenyl)triazine,
Drometrizole trisiloxane,

Polysilicone-15,
1,1-Dicarboxy(2,2'-dimethylpropyl)-4,4-diphenylbutadiene,
2,4-Bis[4[5-(1,1-dimethylpropyl)benzoxazol-2-yl]phenylimino]-6-[(2-ethylhexyl)imino-1,3,5-triazine,
and mixtures thereof.

The particularly preferred organic screening agents are chosen from:

Ethylhexyl salicylate,
Homosalate,
Butylmethoxydibenzoylmethane,
Octocrylene,
n-Hexyl 2-(4-diethylamino-2-hydroxybenzoyl)benzoate,
Terephthalylidene Dicamphor Sulfonic Acid,
Bis(ethylhexyloxyphenol)methoxyphenyltriazine,
Ethylhexyl Triazone,
Diethylhexyl Butamido Triazone,
2,4-Bis(n-butyl 4'-aminobenzalmalonate)-6-[(3-{1,3,3,3-tetramethyl-1-[(trimethylsilyl)oxy]disiloxanyl}propyl)amino]-s-triazine,
Drometrizole Trisiloxane,
and mixtures thereof.

The mineral UV-screening agents used in accordance with the present invention are metal oxide pigments. More preferentially, the mineral UV-screening agents are metal oxide particles with a mean elementary particle size of less than or equal to 0.5 μm, more preferentially between 0.005 and 0.5 μm, even more preferentially between 0.01 and 0.2 μm, better still between 0.01 and 0.1 μm and more particularly between 0.015 and 0.05 μm.
They may be selected in particular from titanium oxide, zinc oxide, iron oxide, zirconium oxide and cerium oxide, or mixtures thereof.
Such coated or uncoated metal oxide pigments are described in particular in patent application EP-A-0 518 773. Commercial pigments that may be mentioned include the products sold by the companies Sachtleben Pigments, Tayca, Merck and Degussa.
The metal oxide pigments may be coated or uncoated.
The coated pigments are pigments that have undergone one or more surface treatments of chemical, electronic, mechanochemical and/or mechanical nature with compounds such as amino acids, beeswax, fatty acids, fatty alcohols, anionic surfactants, lecithins, sodium, potassium, zinc, iron or aluminium salts of fatty acids, metal alkoxides (of titanium or aluminium), polyethylene, silicones, proteins (collagen, elastin), alkanolamines, silicon oxides, metal oxides or sodium hexametaphosphate.
The coated pigments are more particularly titanium oxides that have been coated:

- with silica, such as the product Sunveil® from the company Ikeda,
- with silica and iron oxide, such as the product Sunveil F® from the company Ikeda,
- with silica and alumina, such as the products Microtitanium Dioxide MT 500 SA® and Microtitanium Dioxide MT 100 SA® from the company Tayca and Tioveil from the company Tioxide,
- with alumina, such as the products Tipaque TTO-55 (B)® and Tipaque TTO-55 (A)® from the company Ishihara and UVT 14/4 from the company Sachtleben Pigments,
- with alumina and aluminium stearate, such as the products Microtitanium Dioxide MT 100 T®, MT 100 TX®, MT 100 Z® and MT-01® from the company Tayca, the products Solaveil CT-10 W® and Solaveil CT 100® from the company Uniqema and the product Eusolex T-AVO® from the company Merck,
- with silica, alumina and alginic acid, such as the product MT-100 AQ® from the company Tayca,
- with alumina and aluminium laurate, such as the product Microtitanium Dioxide MT 100 S® from the company Tayca,
- with iron oxide and iron stearate, such as the product Microtitanium Dioxide MT 100 F® from the company Tayca,
- with zinc oxide and zinc stearate, such as the product BR 351® from the company Tayca,
- with silica and alumina and treated with a silicone, such as the products Microtitanium Dioxide MT 600 SAS®, Microtitanium Dioxide MT 500 SAS® or Microtitanium Dioxide MT 100 SAS® from the company Tayca,
- with silica, alumina and aluminium stearate and treated with a silicone, such as the product STT-30-DS® from the company Titan Kogyo,
- with silica and treated with a silicone, such as the product UV-Titan X 195® from the company Sachtleben Pigments,
- with alumina and treated with a silicone, such as the products Tipaque TTO-55 (S)® from the company Ishihara or UV Titan M 262® from the company Sachtleben Pigments,

- with triethanolamine, such as the product STT-65-S from the company Titan Kogyo,
- with stearic acid, such as the product Tipaque TTO-55 (C)® from the company Ishihara,
- with sodium hexametaphosphate, such as the product Microtitanium Dioxide MT 150 W® from the company Tayca,
- $TiO_2$ treated with octyltrimethylsilane, sold under the trade name T 805® by the company Degussa Silices,
- $TiO_2$ treated with a polydimethylsiloxane, sold under the trade name 70250 Cardre UF TiO2SI3® by the company Cardre,
- anatase/rutile $TiO_2$ treated with a polydimethylhydrogenosiloxane, sold under the trade name Microtitanium Dioxide USP Grade Hydrophobic® by the company Color Techniques.

Mention may also be made of $TiO_2$ pigments doped with at least one transition metal such as iron, zinc or manganese and more particularly manganese. Preferably, the said doped pigments are in the form of an oily dispersion. The oil present in the oily dispersion is preferably chosen from triglycerides including those of capric/caprylic acids. The oily dispersion of titanium oxide particles may also comprise one or more dispersants, for instance a sorbitan ester, for instance sorbitan isostearate, or a polyoxyalkylenated fatty acid ester of glycerol, for instance TRI-PPG3 myristyl ether citrate and polyglyceryl-3 polyricinoleate. Preferably, the oily dispersion of titanium oxide particles comprises at least one dispersant chosen from polyoxyalkylenated fatty acid esters of glycerol. Mention may be made more particularly of the oily dispersion of $TiO_2$ particles doped with manganese in capric/caprylic acid triglycerides in the presence of TRI-PPG-3 myristyl ether citrate and polyglyceryl-3 polyricinoleate and sorbitan isostearate having the INCI name: titanium dioxide (and) TRI-PPG-3 myristyl ether citrate (and) polyglyceryl-3 ricinoleate (and) sorbitan isostearate, for instance the product sold under the trade name Optisol TD50® by the company Croda.

The uncoated titanium oxide pigments are sold, for example, by the company Tayca under the trade names Microtitanium Dioxide MT 500 B or Microtitanium Dioxide MT 600 B®, by the company Degussa under the name P 25, by the company Wackher under the name Transparent titanium oxide PW®, by the company Miyoshi Kasei under the name UFTR®, by the company Tomen under the name ITS® and by the company Tioxide under the name Tioveil AQ®.

The uncoated zinc oxide pigments are for example:

- those sold under the name Z-Cote by the company Sunsmart;
- those sold under the name Nanox® by the company Elementis;
- those sold under the name Nanogard WCD 2025® by the company Nanophase Technologies.

The coated zinc oxide pigments are for example:

- those sold under the name Zinc Oxide CS-5® by the company Toshibi (ZnO coated with polymethylhydrogenosiloxane);
- those sold under the name Nanogard Zinc Oxide FN® by the company Nanophase Technologies (as a 40% dispersion in Finsolv TN®, $C_{12}$-$C_{15}$ alkyl benzoate);
- those sold under the name Daitopersion Zn-30® and Daitopersion Zn-50® by the company Daito (dispersions in cyclopolymethylsiloxane/oxyethylenated polydimethylsiloxane, containing 30% or 50% of zinc oxides coated with silica and polymethylhydrogenosiloxane);
- those sold under the name NFD Ultrafine ZnO® by the company Daikin (ZnO coated with perfluoroalkyl phosphate and copolymer based on perfluoroalkylethyl as a dispersion in cyclopentasiloxane);
- those sold under the name SPD-Z1® by the company Shin-Etsu (ZnO coated with silicone-grafted acrylic polymer, dispersed in cyclodimethylsiloxane);
- those sold under the name Escalol Z100® by the company ISP (alumina-treated ZnO dispersed in an ethylhexyl methoxycinnamate/PVP-hexadecene copolymer/methicone mixture);
- those sold under the name Fuji ZnO-SMS-10® by the company Fuji Pigment (ZnO coated with silica and polymethylsilsesquioxane);
- those sold under the name Nanox Gel TN® by the company Elementis (ZnO dispersed at a concentration of 55% in $C_{12}$-$C_{15}$ alkyl benzoate with hydroxystearic acid polycondensate).

The uncoated cerium oxide pigments may be, for example, those sold under the name Colloidal Cerium Oxide® by the company Rhone-Poulenc.

The uncoated iron oxide pigments are sold, for example, by the company Arnaud under the names Nanogard WCD 2002® (FE 45B®), Nanogard Iron FE 45 BL AQ®, Nanogard FE 45R AQ® and Nanogard WCD 2006® (FE 45R®) or by the company Mitsubishi under the name TY-220®.

The coated iron oxide pigments are sold, for example, by the company Arnaud under the names Nanogard WCD 2008 (FE 45B FN)®, Nanogard WCD 2009® (FE 45B 556®), Nanogard FE 45 BL 345® and Nanogard FE 45 BL® or by the company BASF under the name Transparent Iron Oxide®.

Mention may also be made of mixtures of metal oxides, in particular of titanium dioxide and of cerium dioxide, including the equal-weight mixture of titanium dioxide and cerium dioxide coated with silica, sold by the company Ikeda under the name Sunveil A®, and also the mixture of titanium dioxide and zinc dioxide coated with alumina, silica and silicone, such as the product M 261® sold by the company Sachtleben Pigments, or coated with alumina, silica and glycerol, such as the product M 211® sold by the company Sachtleben Pigments.

According to the invention, coated or uncoated titanium oxide pigments are particularly preferred.

The additional UV-screening agents according to the invention are preferably present in the compositions according to the invention in a proportion ranging from 0.1% to 45% by weight and in particular from 5% to 30% by weight, relative to the total weight of the composition.

## Other additives

The aqueous compositions in accordance with the present invention may also comprise conventional cosmetic adjuvants chosen in particular from organic solvents, ionic or nonionic thickeners, softeners, humectants, opacifiers, stabilizers, emollients, silicones, antifoams, fragrances, preserving agents, anionic, cationic, nonionic, zwitterionic or amphoteric surfactants, active agents, fillers, polymers, propellants, alkalizing agents or acidifiers or any other ingredient commonly used in the cosmetic and/or dermatological field.

Among the organic solvents that may be mentioned are lower alcohols and polyols. These polyols may be chosen from glycols and glycol ethers, for instance ethylene glycol, propylene glycol, butylene glycol, dipropylene glycol or diethylene glycol.

Thickeners that may be mentioned include carboxyvinyl polymers, such as carbopols (carbomers) and the Pemulen products (acrylate/$C_{10}$-$C_{30}$-alkyl acrylate copolymer) (Pemulen TR1® or Pemulen TR2®); polyacrylamides, for instance the crosslinked copolymers sold under the names Sepigel 305® (CTFA name: polyacrylamide/$C_{13-14}$ isoparaffin/Laureth 7) or Simulgel 600 (CTFA name: acrylamide/sodium acryloyldimethyltaurate copolymer/isohexadecane/polysorbate 80) by the company SEPPIC; 2-acrylamido-2-methylpropanesulfonic acid polymers and copolymers, optionally crosslinked and/or neutralized, for instance poly(2-acrylamido-2-methylpropanesulfonic acid) sold by the company Hoechst under the trade name Hostacerin AMPS® (CTFA name: ammonium polyacryloyldimethyl taurate or Simulgel 800® sold by the company SEPPIC (CTFA name: sodium polyacryolyldimethyl taurate/polysorbate 80/sorbitan oleate); copolymers of 2-acrylamido-2-methylpropanesulfonic acid and of hydroxyethyl acrylate, for instance Simulgel NS® and Sepinov EMT 10® sold by the company SEPPIC; cellulose derivatives such as hydroxyethylcellulose; polysaccharides and especially gums such as xanthan gum; water-soluble or water-dispersible silicone derivatives, for instance acrylic silicones, polyether silicones and cationic silicones, and mixtures thereof.

Among the acidifying agents, examples that may be mentioned include mineral or organic acids, for instance hydrochloric acid, orthophosphoric acid, sulfuric acid, carboxylic acids, for instance acetic acid, tartaric acid, citric acid or lactic acid, and sulfonic acids.

Among the basifying agents, examples that may be mentioned include aqueous ammonia, alkali metal carbonates, alkanolamines such as monoethanolamine, diethanolamine and triethanolamine, and also derivatives thereof, sodium hydroxide and potassium hydroxide.

Preferably, the cosmetic composition comprises one or more basifying agents chosen from alkanolamines, in particular triethanolamine, and sodium hydroxide.

In the case of a direct emulsion, the pH of the composition in accordance with the invention is generally between 3 and 12 approximately, preferably between 5 and 11 approximately and even more particularly from 6 to 8.5.

Among the active agents for caring for keratin materials such as the skin, the lips, the scalp, the hair, the eyelashes or the nails, examples that may be mentioned include:

- vitamins and derivatives or precursors thereof, alone or as mixtures;
- antioxidants;
- free-radical scavengers;
- antipollution agents;
- self-tanning agents;
- antiglycation agents;
- calmatives;
- deodorants;
- essential oils;
- NO-synthase inhibitors;
- agents for stimulating the synthesis of dermal or epidermal macromolecules and/or which prevent their decomposition;
- agents for stimulating the proliferation of fibroblasts;

- agents for stimulating the proliferation of keratinocytes;
- muscle relaxants;
- refreshing agents;
- tensioning agents;
- mattifying agents;
- depigmenting agents;
- propigmenting agents;
- keratolytic agents;
- desquamating agents;
- moisturizers;
- antiinflammatory agents;
- antimicrobial agents;
- slimming agents;
- agents acting on the energy metabolism of cells;
- insect repellents;
- substance P or CGRP antagonists;
- hair-loss counteractants;
- antiwrinkle agents;
- antiageing agents.

A person skilled in the art will select the said active principle(s) according to the effect desired on the skin, the hair, the eyelashes, the eyebrows or the nails.

Needless to say, a person skilled in the art will take care to select the abovementioned optional additional compound(s) and/or the amounts thereof such that the advantageous properties intrinsically associated with the compositions in accordance with the invention are not, or are not substantially, adversely affected by the envisaged addition(s).

## GALENICAL FORMS

The compositions according to the invention may be prepared according to the techniques that are well known to those skilled in the art. They may be in the form of a cream or a milk. They may optionally be in the form of a mousse or a spray.

The compositions according to the invention find their application in a large number of treatments, especially cosmetic treatments, for the skin, the lips and the hair, including the scalp, especially for protecting and/or caring for the skin, the lips and/or the hair, and/or for making up the skin and/or the lips.

Another subject of the present invention consists of the use of the compositions according to the invention as defined above for the manufacture of products for the cosmetic treatment of the skin, the lips, the nails, the hair, the eyelashes, the eyebrows and/or the scalp, especially care products, antisun products and makeup products.

The cosmetic compositions according to the invention may be used, for example, as makeup products.

Another subject of the present invention consists of a composition according to the invention as defined above for caring for and/or making up a keratin material, which consists in applying, to the surface of the said keratin material, the said composition.

The cosmetic compositions according to the invention may be used, for example, as care products and/or antisun protection products for the face and/or the body, of liquid to semi-liquid consistency, such as milks, more or less smooth creams, gel-creams, and pastes. They may optionally be packaged in aerosol form and may be in the form of a mousse or a spray.

The compositions according to the invention in the form of vaporizable fluid lotions in accordance with the invention are applied to the skin or the hair in the form of fine particles by means of pressurization devices. The devices in accordance with the invention are well known to those skilled in the art and comprise non-aerosol pumps or "atomizers", aerosol containers comprising a propellant and aerosol pumps using compressed air as propellant. These devices are described in patents US 4 077 441 and US 4 850 517.

The compositions packaged as an aerosol in accordance with the invention generally comprise conventional propellants, such as, for example, hydrofluoro compounds, dichlorodifluoromethane, difluoroethane, dimethyl ether, isobutane, n-butane, propane or trichlorofluoromethane. They are preferably present in amounts ranging from 15% to 50% by weight relative to the total weight of the composition.

## ASSEMBLY

According to another aspect, the invention also relates to a cosmetic assembly comprising:

i) a container delimiting one or more compartment(s), the said container being closed by a closing member and optionally not being leaktight; and
ii) a makeup and/or care composition in accordance with the invention placed inside the said compartment(s).

The container may be, for example, in the form of a jar or a box. The closing member may be in the form of a lid comprising a cap mounted so as to be able to move by translation or by pivoting relative to the container housing the said makeup and/or care composition(s).

The examples that follow serve to illustrate the invention without, however, being limiting in nature. In these examples, the amounts of the composition ingredients are given as % by weight, relative to the total weight of the composition.

**Example A1: Preparation of compound (1)**

(1)

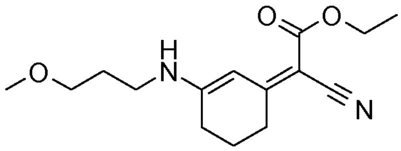

122.23 g of 3-[(3-methoxypropyl)amino]-2-cyclohexen-1-one were alkylated with dimethyl sulfate or alternatively with diethyl sulfate and treated with 75.45 g of ethyl cyanoacetate in approximately equimolar proportions in the presence of a base and optionally of a solvent. The following base/solvent combinations were used:

| Example | Base | Solvent |
|---|---|---|
| Example A1.1 | DBU (1,8-diazabicyclo[5.4.0]undec-7-ene) | dimethylacetamide |
| Example A1.2 | triethylamine | isopropanol |
| Example A1.3 | 3-methoxypropylamine | isopropanol |
| Example A1.4 | 3-methoxypropylamine | tert-amyl alcohol |
| Example A1.5 | 3-methoxypropylamine | toluene |
| Example A1.6 | 3-methoxypropylamine | dimethylformamide |
| Example A1.7 | 3-methoxypropylamine | no solvent |
| Example A1.8 | N-morpholine | isopropanol |

The completion of the alkylation reaction was monitored, for example, via methods such as TLC, GC or HPLC. 162.30 g of compound (14) were obtained in the form of a brown oil. After crystallization, the product was obtained in the form of yellowish crystals.
Melting point: 92.7°C.

**Example A2: Preparation of compound (2)**

(2)

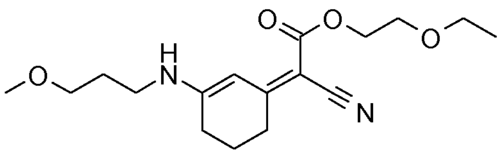

148.4 g of 3-[(3-methoxypropyl)amino]-2-cyclohexen-1-one were alkylated with dimethyl sulfate or alternatively with diethyl sulfate and treated with 130.00 g of 2-ethoxyethyl cyanoacetate in the presence of a base and a solvent. The following base/solvent combinations were used:

| Example | Base | Solvent |
|---|---|---|
| Example A2.1 | DBU (1,8-diazabicyclo[5.4.0]undec-7-ene) | dimethylacetamide |
| Example A2.2 | triethylamine | isopropanol |
| Example A2.3 | 3-methoxypropylamine | isopropanol |
| Example A2.4 | N-methylmorpholine | tert-amyl alcohol |
| Example A2.5 | 3-methoxypropylamine | toluene |
| Example A2.6 | 3-methoxypropylamine | dimethylformamide |
| Example A2.7 | 3-methoxypropylamine | no solvent |

## Formulation Examples 1 to 6

The formulations 1 to 4 below were prepared, in which the chemical stability of compound (2) of the invention was evaluated.

| Phase | Ingredients | Formulation 1 (invention) | Formulation 2 (outside the invention) |
|---|---|---|---|
| A | $C_{12-15}$ ALKYL BENZOATE | 28.72 | - |
| | Compound (2) | 0.5 | 0.5 |
| | PEG-30 DIPOLYHYDROXYSTEARATE (ARLACEL P 135®) | 2 | - |
| | CAPRYLIC/CAPRIC TRIGLYCERIDE | 5 | |
| | CAPRYLYL GLYCOL | 0.5 | - |
| | LAURYL PEG/PPG-18/18 METHICONE (and) DODECENE (and) POLOXAMER 407 | 2 | - |
| | CYCLOHEXASILOXANE | 6 | - |
| | TOCOPHEROL | 0.2 | - |
| | DISTEARDIMONIUM HECTORITE (and) PROPYLENE CARBONATE | 2 | |
| | SILICA | 0.1 | - |
| | PHENETHYL BENZOATE (and) BENZOIC ACID (X-TEND 226® - ISP) | - | 30 |
| | STEARIC ACID | - | 1.5 |
| | GLYCERYL STEARATE (and) PEG-100 STEARATE (ARLACEL 165®) | - | 2.5 |
| | DIMETHICONE | - | 0.5 |
| | CETYL ALCOHOL | - | 0.5 |
| | CETEARYL ALCOHOL (and) CETEARYL GLUCOSIDE (MONTANOV 68® - SEPPIC) | - | 2 |
| | PRESERVING AGENT | - | 1 |

(continued)

| Phase | Ingredients | Formulation 1 (invention) | Formulation 2 (outside the invention) |
|---|---|---|---|
| B | WATER | qs 100 | qs 100 |
| | AMMONIUM POLYACRYLODIMETHYLTAURAMI DE | 0.5 | - |
| | GLYCEROL | 2 | 5 |
| | PROPYLENE GLYCOL | 4 | - |
| | PENTASODIUM ETHYLENEDIAMINE TETRAMETHYLENE PHOSPHONATE | 0.3 | - |
| | TRIETHANOLAMINE | - | 0.45 |
| | DISODIUM EDTA | - | 0.1 |
| | POTASSIUM CETYL PHOSPHATE (AMPHISOL K® -DSM NUTRITIONAL PRODUCTS) | - | 1 |
| C | ISOHEXADECANE | - | 1 |
| | ACRYLATES/C10-30 ALKYL ACRYLATE CROSSPOLYMER (PEMULEN TR1®) | - | 0.2 |
| | XANTHAN GUM | - | 0.2 |
| D | ISODODECANE | 4.5 | - |
| | ALCOHOL DENATURATE | 6 | - |
| | WATER | - | 1.35 |
| | TRIETHANOLAMINE | - | 0.2 |

| Phase | Ingredients | Formulation 3 (outside the invention) | Formulation 4 (invention) |
|---|---|---|---|
| A | Water | qsp 100 | qsp 100 |
| | Glycerin | 5 | 5 |
| | Disodium EDTA | 0.1 | 0.1 |
| | Potassium Cetyl Phosphate (Amphisol K) | 1 | - |
| | Acrylates / C10-30 Alkyl Acrylate crosspolymer | - | 0.2 |
| | Xanthan gum | - | 0.2 |
| | Triethanolamine | - | 0.2 |
| B | ISOPROPYL LAUROYL SARCOSINATE (Eldew SL-205®) | 30 | 30 |
| | Compound (2) | 2 | 2 |
| | POLY C10-30 ALKYL ACRYLATE | 0.5 | 0.5 |
| | Cetyl Alcohol | 0.5 | - |
| | Cetearyl Alcohol (and) Cetearyl Glucoside | 2 | - |
| | PEG-30 DI POLYHYDROXYSTEARATE (Arlacel P135®) | - | 3,5 |
| | Preservatives | 0.9 | 0.9 |
| | Isohexadecane | - | 1 |

(continued)

| Phase | Ingredients | Formulation 3 (outside the invention) | Formulation 4 (invention) |
|---|---|---|---|
| C | Isohexadecane | 1 | - |
| | Acrylates/C10-30 Alkyl Acrylate crosspolymer | 0.2 | - |
| | Xanthan gum | 0.2 | - |
| D | Triethanolamine | 0.2 | - |

Formulations 5 and 6 below were prepared, in which the color was evaluated.

| Phase | Ingrédients | Formule 5 (invention) | Formule 6 (hors invention) |
|---|---|---|---|
| A | ISOPROPYL LAUROYL SARCOSINATE (Eldew SL 205®) | 30 | 30 |
| | Compound 2 | 2 | - |
| | MC11 ofWO2008/090066 | - | 2 |
| | PEG-30 DIPOLYHYDROXYSTEARATE (Arlacel P135®) | 2,5 | 2,5 |
| | Preservative | 0.5 | 0.5 |
| | LAURYL PEG/PPG-18/18 METHICONE (and) DODECENE (and) POLOXAMER 407® | 2 | 2 |
| | DISTEARDIMONIUM HECTORITE (and) PROPYLENE CARBONATE | 2 | 2 |
| B | Water | qsp 100 | qsp 100 |
| | AMMONIUM POLYACRYLOYLDIMETHYL TAURATE (Hostacerin AMPS®) | 0,5 | 0,5 |
| | PROPYLENE GLYCOL | 6 | 6 |
| C | Alcohol | 6 | 6 |

**Method for preparing the emulsions:**

The aqueous phase and oily phase were prepared by mixing the starting materials with mechanical stirring at 80°C. Once the aqueous solution and oily solution were macroscopically homogeneous, the emulsions containing PEG-30 DIPOLYHYDROXYSTEARATE (Arlacel P 135®) were prepared by introducing the aqueous phase into the oily phase, whereas the emulsion containing POTASSIUM CETYL PHOSPHATE (AMPHISOL K®) (outside the invention) was prepared by introducing the oily phase into the aqueous phase, both under stirring using a rotor-stator homogenizer (mixer in the case of the invention). Stirring was maintained for 10 to 20 minutes before adding phase C, then D if it exists, still under stirring.

**Protocol for evaluating the merocyanine stability:**

The stability of the merocyanines in formulation was evaluated by UPLC assay of the residual merocyanine content after 2 months of storage of the formulations at 4°C, at room temperature and at 45°C or after 1 week at 60)C. The percentage of degradation of the merocyanines after 2 months at 45°C or 1 week at 60 °C is expressed as:

$$\text{Degradation}_{\text{t2M45°C}}\ (\%) = \frac{\text{Ct Merocyanine}_{\text{t2M45°C}} - \text{Ct Merocyanine}_{\text{t2MRT°C or 4°C}}}{\text{Ct Merocyanine}_{\text{t2MRT°C or 4°}}} \text{ X } 100$$

$$\text{Degradation}_{t1W60°C}\ (\%) = \frac{\text{Ct Merocyanine}_{t1W60°C} - \text{Ct Merocyanine}_{t0}}{\text{Ct Merocyanine}_{t0}} \times 100$$

t0 corresponds to the initial content introduced in the formulation.

| Evaluated chemical stability | Formulation 1 (invention) | Formulation 2 (outside the invention) |
|---|---|---|
| Residual merocyanine after 2 months at 4°C (%) | - | 0.49 |
| Residual merocyanine after 2 months at RT°C (%) | 0.53 | - |
| Residual merocyanine after 2 months at 45°C (%) | 0.53 | 0.46 |
| Merocyanine degradation after 2 months at 45°C (%) | < 1% | 8% |

| Evaluated chemical stability | Formule 3 (outside the invention) | Formulation 4 (invention) |
|---|---|---|
| Residual merocyanine after 1 week at 60°C (%) | 1.79 | 2.05 |
| Merocyanine degradation after 1 week at 60°C (%) | **10.5** | **< 5%** |

The stability results obtained on formulations 1 and 4 according to the invention in comparison with formulations 2 et 3 show that the merocyanines of the invention, formulated in emulsified compositions of the invention, are chemically more stable than when they are formulated in a support comprising an emulsifying system containing at least one alkali metal salt of a phosphoric acid ester of a fatty alcohol.

**Protocol for the evaluation of the color of the formulations 5 and 6 :**

The color of the formulations was evaluated in a clear glass recipient. « + » denotes a week yellow level and « +++ » denotes a yellow level which is substantially more important to the naked eye.

| Color | Formulation 5 (invention) | Formule 6 (outside the invention) |
|---|---|---|
| Yellow | + | +++ |

The results on the examples 5 and 6 show that the formulation 5 with the compound (2) is significantly less yellow than the equivalent formulation 6 with the compound MC11 of the application WO2008/090066.

## Claims

**1.** Cosmetic or dermatological composition in the form of a water-in-oil emulsion comprising, in a physiologically acceptable support:

a) at least one aqueous phase,
b) at least one oily phase,
c) at least one merocyanine compound of the formula (1) or one of the E/E- or E/Z-geometrical isomer forms thereof:

(1) 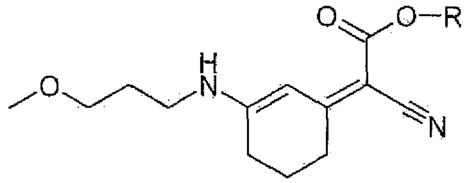

in which:
R is a $C_1$-$C_{22}$ alkyl group, a $C_2$-$C_{22}$ alkenyl group, a $C_2$-$C_{22}$ alkynyl group, a $C_3$-$C_{22}$ cycloalkyl group or a $C_3$-$C_{22}$ cycloalkenyl group, it being possible for the said groups to be interrupted with one or more O and
d) at least one emulsifying system comprising at least one emulsifying polymer of the polyoxyalkylenated glycol fatty acid ester type,
said polymer is a block polymer comprising poly(hydroxylated ester) blocks and polyethylene glycol blocks,
said fatty acid ester of the said emulsifying polymer as defined above bears a chain comprising from 12 to 20 carbon atoms.

2. Composition according to Claim 1, in which the merocyanine compound(s) are chosen from those in which:
R is a $C_1$-$C_{22}$ alkyl, which may be interrupted with one or more O.

3. Composition according to Claim 1 or 2, in which the merocyanine compound(s) are chosen from the following compounds and also the E/E- or E/Z- geometrical isomer forms thereof:

| | | | |
|---|---|---|---|
| 1 | 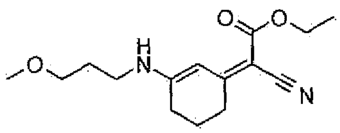 ethyl (2Z)-cyano{3-[(3-methoxypropyl)amino] cyclohex-2-en-1-ylidene}ethanoate | 4 | 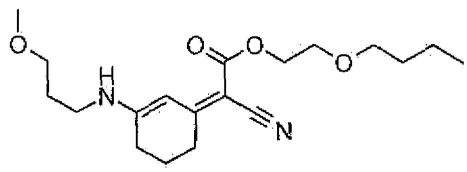 2-butoxyethyl (2Z)-cyano{3-[(3-methoxypropyl) amino]cyclohex-2-en-1-ylidene}ethanoate |
| 2 | 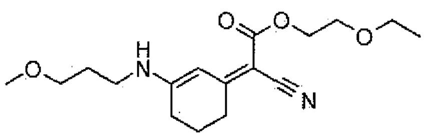 2-ethoxyethyl (2Z)-cyano{3-[(3-methoxypropyl) amino]cyclohex-2-en-1-ylidene}ethanoate | 5 | 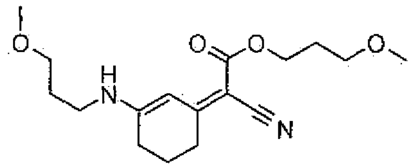 3-methoxypropyl (2Z)-cyano{3-[(3-methoxypropyl) amino]cyclohex-2-en-1-ylidene)ethanoate |
| 3 | 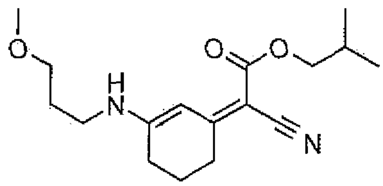 2-methylpropyl(2Z)-cyanof3-[(3-methoxypropyl) amino]cyclohex-2-en-1-ylidene}ethanoate | 6 | 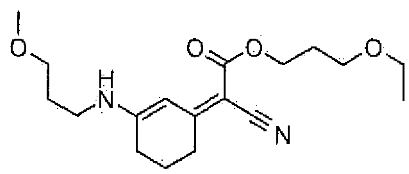 3-ethoxypropyl(2Z)-cyano{3-[(3-methoxypropyl) amino]cyclohex-2-en-1-y)idene}ethanoate |

4. Composition according to Claim 3, in which the merocyanine compound is 2-ethoxyethyl (2Z)-cyano{3-[(3-methoxypropyl)amino]cyclohex-2-en-1-ylidene}ethanoate (2) in its E/Z geometrical configuration having the following structure:

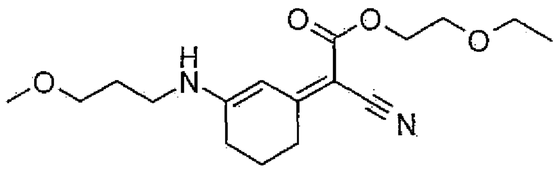

and/or in its E/E geometrical configuration having the following structure:

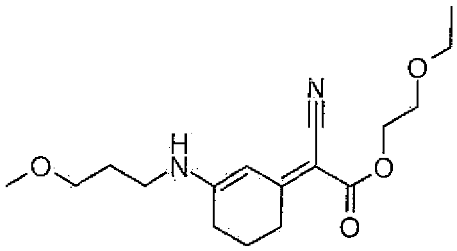

5. Composition according to any one of Claims 1 to 4, in which the merocyanine compound(s) of formula (1) are present in a concentration ranging from 0.1% to 10% by weight and preferentially from 0.2% to 5% by weight relative to the total weight of the composition.

6. Composition according to any one of Claims 1 to 5, in which the said emulsifying polymer is a block polymer of structure ABA comprising poly(hydroxylated ester) blocks and polyethylene glycol blocks.

7. Composition according to any one of the preceding claims, in which the fatty acid ester of the said emulsifying polymer has a chain comprising from 14 to 18 carbon atoms.

8. Composition according to any one of the preceding claims, in which the polyethylene glycol blocks of the said emulsifying polymer comprise from 4 to 50 mol of ethylene oxide and preferably from 20 to 40 mol of ethylene oxide.

9. Composition according to any one of the preceding claims, in which the said emulsifying polymer is a polyethylene glycol dipolyhydroxystearate comprising 30 mol of ethylene oxide.

10. Composition according to any one of the preceding claims, in which the emulsifying polymer(s) are present in a content ranging from 0.2% to 10% by weight and more preferentially from 0.25% to 5% by weight relative to the total weight of the composition.

11. Composition as defined in any one of the preceding claims for caring for and/or making up a keratin material, comprising the application, to the surface of the said keratin material of the said composition.

12. Composition as defined in any one of the preceding claims for limiting the darkening of the skin and/or improving the colour and/or uniformity of the complexion, comprising the application, to the surface of the skin of the said composition.

13. Composition as defined in any one of the preceding claims for preventing and/or treating the signs of ageing of a keratin material, comprising the application, to the surface of the keratin material of the said composition.

## Patentansprüche

1. Kosmetische oder dermatologische Zusammensetzung in Form einer Wasser-in-Öl-Emulsion, umfassend in einem physiologisch unbedenklichen Träger:

a) mindestens eine wässrige Phase,
b) mindestens eine ölige Phase und
c) mindestens eine Merocyanin-Verbindung der Formel (1) oder eine der geometrischen E/E- oder E/Z-Isomer-

formen davon:

(1) 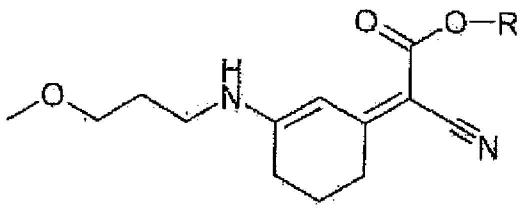

wobei:
R für eine $C_1$-$C_{22}$-Alkylgruppe, eine $C_2$-$C_{22}$-Alkenylgruppe, eine $C_2$-$C_{22}$-Alkinylgruppe, eine $C_3$-$C_{22}$-Cycloalkylgruppe oder eine $C_3$-$C_{22}$-Cycloalkenylgruppe steht, wobei die Gruppen gegebenenfalls durch ein oder mehrere 0 unterbrochen sein können, und
d) mindestens ein Emulgiersystem, das mindestens ein emulgierendes Polymer vom Typ polyoxyalkylenierter Glykolfettsäureester umfasst,
wobei es sich bei dem Polymer um ein Blockcopolymer mit Poly(hydroxyester)-Blöcken und Polyethylenglykol-Blöcken handelt,
wobei der Fettsäureester des emulgierenden Polymers gemäß obiger Definition eine Kette mit 12 bis 20 Kohlenstoffatomen trägt.

**2.** Zusammensetzung nach Anspruch 1, wobei die Merocyanin-Verbindung(en) aus denjenigen ausgewählt ist bzw. sind, in denen:

R für ein $C_1$-$C_{22}$-Alkyl, das durch ein oder mehrere O unterbrochen sein kann,
steht.

**3.** Zusammensetzung nach Anspruch 1 oder 2, wobei die Merocyanin-Verbindung(en) aus den folgenden Verbindungen sowie den geometrischen E/E- oder E/Z-Isomerformen davon ausgewählt ist bzw. sind:

| | | | |
|---|---|---|---|
| 1 | 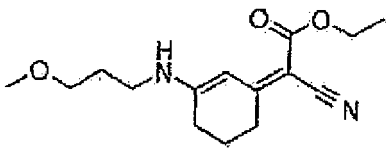 (2Z)-Cyano{3-[(3-methoxypropyl) amino]-cyclohex-2-en-1-yliden}ethansäure-ethylester | 4 | 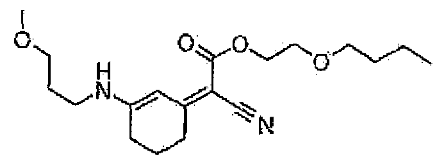 (2Z)-Cyano{3-[(3-methoxypropyl)amino]-cyclohex-2-en-1-yliden}ethansäure-2-butoxyethylester |
| 2 | 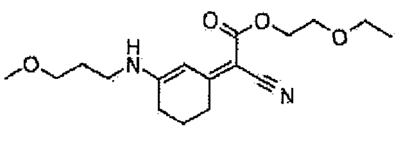 (2Z)-Cyano{3-[(3-methoxypropyl) amino]-cyclohex-2-en-1-yliden}ethansäure-2-ethoxyethylester | 5 | 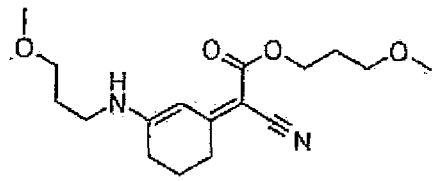 (2Z)-Cyano{3-[(3-methoxypropyl)amino]-cyclohex-2-en-1-yliden}ethansäure-3-methoxypropylester |

(fortgesetzt)

| | | | |
|---|---|---|---|
| 3 | 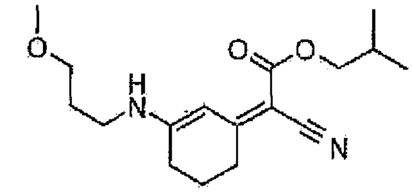<br>(2Z)-Cyano{3-[(3-methoxypropyl) amino]-cyclohex-2-en-1-yliden}ethansäure-2-methylpropylester | 6 | 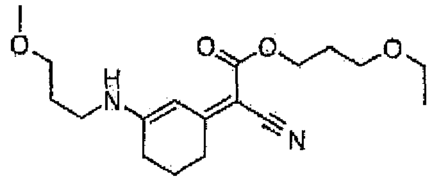<br>(2Z)-Cyano{3-E(3-methoxypropyl)amino]-cyclohex-2-en-1-yliden}ethansäure-3-ethoxypropylester |

4. Zusammensetzung nach Anspruch 3, wobei es sich bei der Merocyanin-Verbindung um (2Z)-Cyano{3-[{3-methoxypropyl)Amino]cyclohex-2-en-1-yliden)ethansäure-2-ethoxyethylester (2) in seiner geometrischen E/Z-Konfiguration mit der folgenden Struktur:

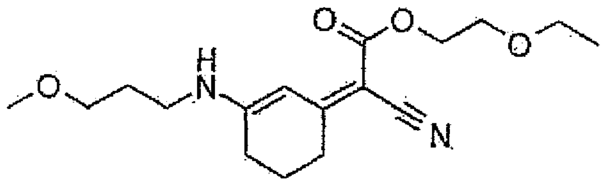

und/oder in seiner geometrischen E/E-Konfiguration mit der folgenden Struktur:

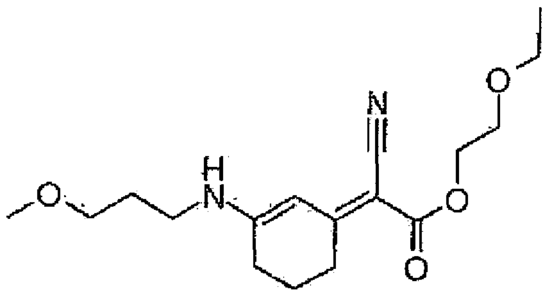

handelt.

5. Zusammensetzung nach einem der Ansprüche 1 bis 4, wobei die Merocyanin-Verbindung (en) der Formel (1) in einer Konzentration im Bereich von 0,1 bis 10 Gew.-% und vorzugsweise von 0,2 bis 5 Gew.-%, bezogen auf das Gesamtgewicht der Zusammensetzung, vorliegt bzw. vorliegen.

6. Zusammensetzung nach einem der Ansprüche 1 bis 5, wobei es sich bei dem emulgierenden Polymer um ein Blockcopolymer mit ABA-Struktur, das Poly (hydroxyester)-Blöcke und Polyethylenglykol-Blöcke umfasst, handelt.

7. Zusammensetzung nach einem der vorhergehenden Ansprüche, wobei der Fettsäureester des emulgierenden Polymers eine Kette mit 14 bis 18 Kohlenstoffatomen aufweist.

8. Zusammensetzung nach einem der vorhergehenden Ansprüche, wobei die Polyethylenglykol-Blöcke des emulgierenden Polymers 4 bis 50 mol Ethylenoxid und vorzugsweise 20 bis 40 mol Ethylenoxid umfassen.

9. Zusammensetzung nach einem der vorhergehenden Ansprüche, wobei es sich bei dem emulgierenden Polymer um ein Polyethylenglykoldihydroxystearat mit 30 mol Ethylenoxid handelt.

10. Zusammensetzung nach einem der vorhergehenden Ansprüche, bei dem das emulgierende Polymer bzw. die emulgierenden Polymere in einem Gehalt im Bereich von 0,2 bis 10 Gew.-% und weiter bevorzugt von 0,25 bis 5 Gew.-%, bezogen auf das Gesamtgewicht der Zusammensetzung, vorliegt bzw. vorliegen.

**11.** Zusammensetzung gemäß einem der vorhergehenden Ansprüche zum Pflegen und/oder Schminken eines Keratinmaterials, wobei man die Zusammensetzung auf die Oberfläche des Keratinmaterials aufbringt.

**12.** Zusammensetzung gemäß einem der vorhergehenden Ansprüche zum Einschränken des Dunkelwerdens der Haut und/oder zum Verbessern der Farbe und/oder Einheitlichkeit des Teints, wobei man die Zusammensetzung auf die Oberfläche der Haut aufbringt.

**13.** Zusammensetzung gemäß einem der vorhergehenden Ansprüche zum Verhindern und/oder Behandeln der Anzeichen von Alterung eines Keratinmaterials, wobei man die Zusammensetzung auf die Oberfläche des Keratinmaterials aufbringt.

## Revendications

**1.** Composition cosmétique ou dermatologique sous forme d'émulsion eau-dans-huile comprenant dans un support physiologiquement acceptable:

a) au moins une phase aqueuse,
b) au moins une phase huileuse,
c) au moins un composé mérocyanine de formule (1) ou l'une de ses formes géométriques isomères E/E- ou E/Z-:

(1) 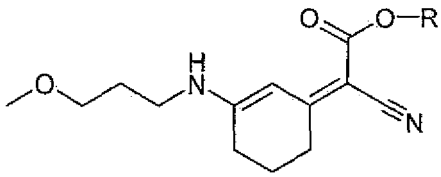

dans laquelle
R est un groupement alkyle en $C_1$-$C_{22}$, un groupement alcényle en $C_2$-$C_{22}$, un groupement alcinyle en $C_2$-$C_{22}$, un groupement cycloalkyle en $C_3$-$C_{22}$ ou un groupement cycloalcényle en $C_3$-$C_{22}$, lesdits groupements pouvant être interrompus par un ou plusieurs O et
d) au moins un système émulsionnant contenant au moins un polymère émulsionnant du type ester d'acide gras et de glycol polyoxyalkyléné,
ledit polymère est un polymère séquence comportant des séquences poly(ester hydroxylé) et des séquences polyéthylèneglycol,
ledit ester d'acide gras dudit polymère émulsionnant tel que défini précédemment porte une chaîne comportant de 12 à 20 atomes de carbone.

**2.** Composition selon la revendication 1, où le ou les composés mérocyanine sont choisis parmi ceux où R est un alkyle en $C_1$-$C_{22}$, pouvant être interrompu par un ou plusieurs O.

**3.** Composition selon la revendication 1 ou 2, où le ou les composés mérocyanine sont choisis parmi les composés suivants ainsi que leurs formes géométriques isomères E/E- ou E/Z-:

| | | | |
|---|---|---|---|
| 1 | 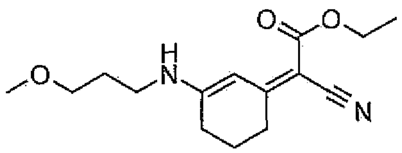 ethyl (2Z) -cyano{3-[(3-methoxypropyl)amino] cyclohex -2-en-1-ylidene}ethanoate | 4 | 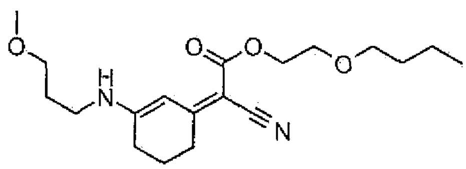 2-butoxyethyl (2Z)-cyano{3-[(3-methoxypropyl) amino]cyclohex -2-en-1-ylidenelethanoate |

(suite)

| | | | |
|---|---|---|---|
| 2 | 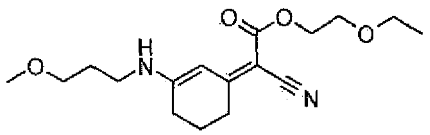<br>2-ethoxyethyl (2Z)-cyano{-3-[(3-methoxypropyl) amino]cyclohex -2-en-1-ylidene}ethanoate | 5 | 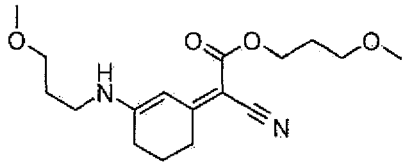<br>3-methoxypropyl (2Z)-cyano{3-[(3-methoxypropyl) amino]cyclohex -2-en-1-ylidene}ethanoate |
| 3 | 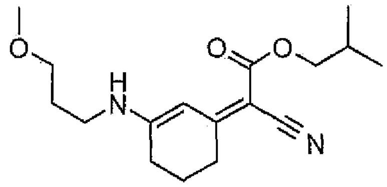<br>2-methylpropyl (2Z)-cyano{3-E(3-methoxypropyl) amino]cyclohex -2-en-1-ylidene}ethanoate | 6 | 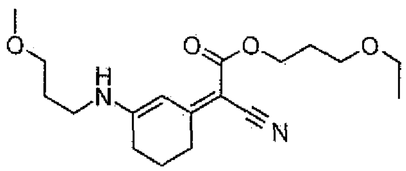<br>3-ethoxypropyl (2Z)-cyano{3-[(3-methoxypropyl) amino]cyclohex -2-en-1-ylidene}ethanoate |

4. Composition selon la revendication 3, où le composé mérocyanine est le 2-éthoxyéthyle (2Z)-cyano{3-[(3-méthoxypropyl)-aminolcyclohex-2-èn-1-ylidène}éthanoate (2) dans sa configuration géométrique E/Z de structure suivante :

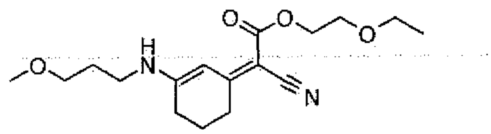

et/ou dans sa configuration géométrique E/E de structure suivante :

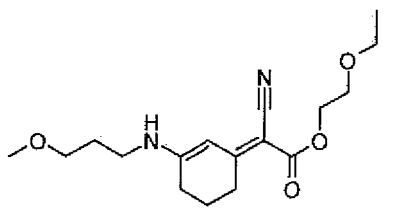

5. Composition selon l'une quelconque des revendications 1 à 4, où le ou les composés mérocyanine de formule (1) sont présents dans une concentration allant de 0,1% à 10% en poids, et préférentiellement de 0,2% à 5% en poids par rapport au poids total de la composition.

6. Composition selon l'une quelconque des revendications 1 à 5, où ledit polymère émulsionnant est un polymère séquencé de structure ABA comportant des séquences poly(ester hydroxylé) et des séquences polyéthylèneglycol.

7. Composition selon l'une quelconque des revendications précédentes, où l'ester d'acide gras dudit polymère émulsionnant présente une chaîne comportant de 14 à 18 atomes de carbone.

8. Composition selon l'une quelconque des revendications précédentes, où les séquences polyéthylèneglycol dudit polymère émulsionnant comportent de 4 à 50 moles d'oxyde d'éthylène, et de préférence de 20 à 40 moles d'oxyde d'éthylène.

9. Composition selon l'une quelconque des revendications précédentes, où ledit polymère émulsionnant est un di-polyhydroxystéarate de polyéthylène glycol comportant 30 moles d'oxyde d'éthylène.

10. Composition selon l'une quelconque des revendications précédentes, où le ou les polymères émulsionnants sont présents à une teneur allant de 0,2 % à 10 % en poids, et plus préférentiellement de 0,25 % à 5 % en poids, par

rapport au poids total de la composition.

**11.** Composition telle que définie dans l'une quelconque des revendications précédentes pour le soin et/ou le maquillage d'une matière kératinique comprenant l'application sur la surface de ladite matière kératinique de ladite composition.

**12.** Composition telle que définie dans l'une quelconque des revendications précédentes pour limiter l'assombrissement de la peau et/ou améliorer la couleur et/ou l'homogénéité du teint comprenant l'application sur la surface de la peau de ladite composition.

**13.** Composition telle que définie dans l'une quelconque des revendications précédentes pour prévenir et/ou traiter les signes du vieillissement d'une matière kératinique comprenant l'application sur la surface de la matière kératinique de ladite composition.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4195999 A
- WO 2004006878 A
- WO 2008090066 A
- WO 2011113718 A
- WO 2009027258 A
- WO 2013010590 A
- WO 2013011094 A
- WO 2013011480 A
- WO 2007071582 A
- US 4749643 A
- WO 2007068371 A
- WO 2008155059 A
- US 5624663 A
- EP 669323 A
- US 2463264 A
- US 5237071 A
- US 5166355 A
- GB 2303549 A
- DE 19726184
- EP 893119 A
- EP 0832642 A
- EP 1027883 A
- EP 1300137 A
- DE 10162844
- WO 9304665 A
- DE 19855649
- EP 0967200 A
- DE 19746654
- DE 19755649
- EP 1008586 A
- EP 1133980 A
- EP 133981 A
- WO 2007071584 A
- WO 2009063392 A
- US 6225467 B
- WO 2004085412 A
- WO 06035000 A
- WO 06034982 A
- WO 06034991 A
- WO 06035007 A
- WO 2006034992 A
- WO 2006034985 A
- EP 0841341 A
- EP 0518773 A
- US 4077441 A
- US 4850517 A


### Non-patent literature cited in the description


- *IP COM JOURNAL N°000179675D,* 23 February 2009
- *IP COM JOURNAL N°000182396D*
- *IP COM JOURNAL N° 000189542D,* 12 November 2009
- *IP COM Journal N°IPCOM000011179D,* 03 April 2004
- **W.C. GRIFFIN.** Classification of Surface Active Agents by HLB. *Journal of the Society of Cosmetic Chemists,* 1949, vol. 1, 311
- Calculation of HLB of Non Ionic Surfactants. *Journal of the Society of Cosmetic Chemists,* 1954, vol. 5, 249
- *CHEMICAL ABSTRACTS,* 919803-06-8
- Symmetrical Triazine Derivatives. *IP.COM IPCOM000031257 Journal, INC,* 20 September 2004